Europäisches Patentamt

European Patent Office

Office européen des brevets

(11) Publication number: **0 067 130**
**B1**

(12) **EUROPEAN PATENT SPECIFICATION**

(45) Date of publication of patent specification: 25.06.86

(21) Application number: 82830117.6

(22) Date of filing: 29.04.82

(51) Int. Cl.⁴: **A 61 K 39/35,** A 61 K 9/48,
A 61 K 9/72

(54) **An allergen dry powder composition and process for its preparation.**

(30) Priority: 04.06.81 IT 2213681

(43) Date of publication of application:
15.12.82 Bulletin 82/50

(45) Publication of the grant of the patent:
25.06.86 Bulletin 86/26

(84) Designated Contracting States:
AT BE DE LU NL SE

(56) References cited:
GB-A-1 055 465
GB-A-1 247 614
GB-A-1 318 560
GB-A-1 381 872

T.M. FEINBLATT et al.: "Annals of allergy", vol.
7, 1949, page 228

(73) Proprietor: **LABORATORIO FARMACEUTICO
LOFARMA S.a.s.**
**Viale Cassala, 40**
**I-20143 Milano (IT)**
(73) Proprietor: **Melillo, Gaetano**
**Via Santo Strato, 7**
**I-80123 Napoli (IT)**

(72) Inventor: **Melillo, Gaetano**
**Via Santo Strato, 7**
**I-80123 Napoli (IT)**

(74) Representative: **Cicogna, Franco**
**Ufficio Internazionale Brevetti Dott.Prof. Franco
Cicogna Via Visconti di Modrone, 14/A**
**I-20122 Milano (IT)**

## Description

This invention concerns capsules containing the active principle of an allergen, and the process for their preparation.

In particular, this invention concerns capsules, which contain the active principle of an allergen, and which are particularly suitable to be administered by inhalation.

As everybody knows, when a patient, who suffers from asthma or allergic rhinitis, inhales through his respiratory system the substance to which he is sensitive, a cell reaction takes place. This reaction leads to the degranulation of the mast cells and to the release in the tissues of substances, which trigger off the obstruction of the respiratory system, bronchial spasm and/or rhinitis. In order to avoid or limit this cell reaction, a therapy has been carried on, in these last few years, with great success. It is the specific hyposensitization, which consists, as everybody knows, in administering to the patient increasing doses of the allergen to which he is sensitive.

The administration of the allergen is generally carried out either parenterally or by atomization and inhalation of an aqueous solution of the allergen. Unfortunately, there are serious drawbacks to both types of administration.

As far as hyposensitization therapy by parenteral administration is concerned, the main drawbacks are:

a) systemic reactions, which could even lead to an anaphylactic shock, might occur, owing to a mistake in the dosage, or to an imperfect execution, or to an abnormal biological reactivity of the patient;

b) the administration must be absolutely made under medical control, or better under the control of the specialist in allergology because of its well known potentially dangerous reaction;

c) the parenterally administered allergen does not get directly to the target organ where the pathological process takes place.

As far as the administration of the allergen by inhalation is conerned, even if we cannot deny its satisfactory results in the reduction of bronchial reactivity in some asthmatic patients, we must point out the following drawbacks: first of all, it implies the use of bulky and specialized equipment (atomizer) and, moreover, it causes a reduction in the activity of the allergen due to its frequent instability in aqueous solution. Moreover, the administration of raw material, e.g. culture in the case of mites, presents disadvantages as well: the administration of raw material is difficult in itself; the quantity of the active principle is variable; the presence of extraneous material, for example culture medium, can involve unwanted and uncontrollable phenomena, such as non specific irritation.

It is also known in the art to administrate powdered mixtures of an allergen and an excipient, but the disclosed mixtures are never dry, uniform, homogenous and alone.

Thus, T. M. Feinblatt et al. "Annals of allergy", Vol 7, 1949, pages 228—231 discloses capsules containing a mixture of pollens and a filler, suitable for oral administration. In the mixture, however, the pollens are not uniformly and homogeneously diluted throughout the filler.

Wet powder, granules or tablets of a mite antigen, suitable to be injected in the form of a solution in an inert pharmaceutically acceptable solvent is known from GB—A—1 318 560. According to Example 9 of this reference, if it is desired to prepare the solid dispensing composition in form of a powder, then the mixture of mite antigen and diluent is ground to a uniform particle size of between No. 40 and No. 60 standard mesh size. The mixture thus obtained, however, does not result to be perfectly homogeneous and uniform.

A pharmaceutical powder composition comprising a mixture of a solid inhalation medicament and a solid carrier is known from GB—A—1 381 872. This reference deals with medicaments such as antianaphylactic agents and not with allergens.

GB—A—1 055 465 discloses intimate and uniform mixtures of an allergenic material in the presence of a surfactant and an aerosol propellent.

GB—A—1 247 614 discloses pure powdered allergen preparation but no mixture thereof with a powdered excipient.

In accordance with this invention, allergen dry powder compositions comprising a uniform and homogeneous mixture of the active principle of the allergen and one or more excipients are provided by wet or solution mixing the active principle with the excipient(s).

The processes as claimed in claims 5 and 6 are especially suitable for the preparation of such allergen dry powder compositions.

The allergen dry powder composition may be contained in shelled or hard cased capsules which are administered by a normal inhaler or turbo inhaler, many types of which are commonly available on the market. The capsules can be used both for therapeutic and diagnostic purposes, that is provocation tests.

The active principle of an allergen can be prepared by extracting the pure raw material in aqueous solutions. Temperature, pH value, extracting time, saline concentration of the solution during the extraction process change from allergen to allergen. For example, as far as mites are concerned, it can be far more advantegeous to carry out the extraction at pH 7.2, at a temperature of +4°C, at a concentration of the solution, containing monobasic and dibasic sodium phosphate, equivalent to 0.15 M, and to stir it for 24 hours.

The active principle of the allergen dissolves in aqueous solutions and, after subsequent centrifugations and filtrations, it is a limpid and homogeneous extract, which can be dehydrated, for example by freeze-drying.

The active principle of any allergen can be used to prepare the dry powder compositions of this invention.

Just to give some examples, we mention mite allergens, such as Dermatophagoides, pollen allergens, animal danders and moulds. The excipients can be selected within a wide range of known compounds, provided that they will not cause, as far as they are concerned, bronchoconstriction, irritating actions on the nasal mucous membrane, and other side-effects.

Excipients, which can be advantageously used in the preparation of the capsules, are: glucose, lactose and saccharose. In addition to or in replacement of the excipient, it is also possible to add to allergens one or more substances, which have a medical activity.

As far as capsules are concerned, they are preferably of the hard type, and are also called shelled capsules.

Normally they consist of gelatine, moulded in such a way as to form two cylinders both closed at one end and perfectly fitting the one within the other, thus delimiting a cavity in which the medicine or the excipient, containing the medicine, which in our case corresponds to the active principle of one or more allergens, remains enclosed.

It is also possible to use capsules consisting of different materials, provided that they have similar characteristics and they are suitable for the use they are intended for. In fact, in practice, according to this invention, the capsules act only as containers. Therefore it is not necessary that they consist of substances assimilable by the organism.

In the capsules, we are speaking of, the content of the allergen active priciple can vary within a wide range: generally small quantities, ranging from some thousanths to some milionths grams per dose, are enough to obtain the desired effect. The quantities of allergens, used in practice, vary in the range mentioned above, according to the type of allergen, object of the therapy, and what stage of therapy the patient has reached, as the hyposensitization therapy generally needs the adminstration of progressively increasing quantities of allergen. The excipient quantity to use is not critical and can vary a lot according to the allergen: for example between 1:1 and 50,000:1.

Tests performed by the Applicants have established that to obtain positive and constant results hyposensitization, using the capsules of this invention, it is necessary, first of all, to use the active principle of the allergen and, moreover, that the dilution of the allergen in the excipient is homogeneous, that is to say that the allergen must be uniformly diluted in the excipient mass. So, for example, if the dilution of the allergen in the excipient is carried out by stirring the dry-state components, thinly subdivided, even if the stirring lasts a very long period (24 hours or more), it can happen that the mixture is not perfectly homogeneous.

This can involve that the organic reactions of the patient to the inhalation of the same quantity of allergen-excipient mixture vary from time to time, and that, moreover the patient shows anomalous and unwanted reactions, due to the inhalation of a greater quantity of allergen than necessary.

From this point of view even the process for the preparation of the dilution of the allergen in the excipient has a great importance.

The methods to prepare the allergen-excipient dilution, which allowed the Applicants to obtain optimum results in terms of activity and constant yield of the product, are the following ones.

Method (A)

An aqueous solution of the active principle of the allergen is diluted, under stirring at room temperature, in the excipient mass, for example lactose, in such a quantity as to cause neither dissolution of the excipient or the formation of a wet mass, but rather the formation of a moist mass with the aspect and the consistency of powder.

Satisfactory results have been obtained diluting 125 ml of the aqueous solution of the active principle with 1000 grams of powdered lactose.

If the allergen is in the dry state, for example freeze-dried, it is diluted with a similar quantity of water.

The moist mass, so obtained, is accurately mixed and homogenised by means of repeated passages through a sieve with a rather large mesh size, for example a sieve with a mesh size of 500 μm.

The mass, so obtained, is dried at a temperature such as not to alter the product (30°—50°C), for example through drying in a air-current oven. The powdered mass is reduced to a fine powder by means of further passages through sieves with smaller and smaller meshes, for example by means of successive passages through stainless steel sieves with a mesh size of 180, 100 and 50 μm.

The product, so obtained, is distributed into rigid gelatine capsules, for example in quantity of 50 mg per capsule.

Method (B)

The excipient is added to 6 l water. To the resulting solution is added the active principle of the allergen, either in solution or dehydrated, for example freeze-dried. The mixture is stirred for a long time (1—10 hours), till it reaches a perfect homogeneity. The solution so obtained is then dehydrated, preferably through freeze-drying.

The anhydrous mass so obtained is crushed in a mortar and then reduced to a fine powder either by repeated passages through sieves with progressively smaller meshes, as described in process (A) or by means of a micronizer, for example one in which the break up of the material is obtained by a fluid jet mill.

Using a micronizer, it is possible to obtain a powder consisting of lactose and allergen, whose particles have the following dimensions: 90% are smaller than 10 μm and the remaining 10% smaller that 20 μm.

The finely powdered material so obtained is distributed into rigid gelatine capsules, for example in quantity of 50 mg per capsule.

The administration to the patient of the active principle of the allergen, which is inside the capsules, can be effected by inserting the capsules in any one of the known bronchial inhalers or nasal sprays. The functioning of these inhalers and sprays varies from type to type, but it generally lies in the perforation of the capsule and in the emission of the powder through the holes, due to the suitable air flows.

The following example aims to illustrate better the inventive concept of this invention without, however, limiting it.

Example

A mixture of pure culture of Dermatophagoides is prepared in a solution of monobasic and dibasic sodium phosphate, with a concentration equivalent to 0.15 molar, buffered at pH 7.2. The mixture is kept at +4°C under stirring for 24 hours. After repeated centrifugations and filtrations of the aqueous mixture, a limpid and homogeneous extract is obtained.

125 ml of this extract, containing 2 mg/ml of active principle, are diluted under stirring in 1000 g of powdered lactose.

The material is mixed till a homogeneous moist powder is obtained. This moist powder is passed through sieves with meshes with a size of 500 μm and the granulate, so obtained, is dried in an air current oven at 35°C for a whole night. This mixture is, then, passed through progressively smaller meshes of 180, 100 and 50 μm.

This finely powdered material is distributed into rigid gelatine capsules in quantity of 50 mg per capsule.

On the basis of a titration of the allergenic content, according to the RAST-inhibition technique (see method of Yman L. and coll., Develop. Biol. Standard., Karger Basel, Vol. 29, pages 151—165, 1975), it has been found that the difference between the allergenic content of various capsules is very small (less than 10%) and such content corresponds to that of the originary material.

The capsule is put in a commercially available inhaler tube and is inhaled by the patient, after the opening of the capsule upon operation of the device contained in the inhaler itself.

The admistration of the product to several patients, all sensitive to the tested allergen, has never triggered off any anomalous bronchobstructive phenomena even after numerous administrations.

**Claims**

1) An allergen dry powder composition comprising a mixture of the active principle of the allergen and one or more excipients, characterized in that the dilution of the active principle of the allergen in the excipient is uniform and homogeneous by wet or solution mixing the active principle with the excipient.

2) An allergen dry powder composition according to claim 1, characterized in that it is contained in hard cased capsules.

3) An allergen dry powder composition according to claim 1 or 2, characterized in that the weight ratio between the excipient and the active principle of the allergen ranges from 1:1 to 50,000:1.

4) An allergen dry powder composition according to any of the preceding claims, characterized in that the particle size of the powder is from 2 to 100 μm.

5) A process for the preparation of the dry allergen powder composition according to any of the preceding claims, consisting in extracting the active principle of the allergen by aqueous extraction, in mixing the active principle with the excipient and grounding the mixture to a uniform particle size, characterized in that the mixing of the active principle with the excipient is carried out by adding the aqueous solution of the active principle of the allergen to the excipient in such quantity to form a moist mass, mixing and homogenizing the moist mass so obtained by repeated passages through a first sieve preferably of 500 μm mesh size, drying the homogenized mass so obtained, reducing the powder mass to a fine powder by means of repeated passages through sieves of progressively smaller mesh size, the last sieve having a 50 μm mesh size, whereby a powder containing the allergen uniformly dispersed in the excipient is obtained.

6) A process for the preparation of the dry allergen powder composition according to claim 5, characterized in that the mixing of the active principle with the excipient is obtained by adding the excipient to water, adding to the solution so obtained the active principle of the allergen in the form of a solution or dehydrated powder, stirring the mixture until perfect homogeneity is obtained, dehydrating the homogeneous solution through freeze-drying, crushing the anydrous mass and powdering the crushed mass either by means of repeated passages through sieves with progressively smaller mesh dimensions or by means of micronisation.

**Patentansprüche**

1. Trockene pulverige Allergenzusammensetzung aus einem Gemisch des aktiven Prinzips des Allergens und einem oder mehreren Bindemitteln, dadurch gekennzeichnet, dass die Verdünnung des aktiven Prinzips des Allergens in dem Bindemittel gleichförmig und homogen erhalten wird, indem man das aktive Prinzip mit dem Bindemittel feucht oder in einer Lösung mischt.

2. Trockene pulverige Allergenzusammensetzung nach Anspruch 1, dadurch gekennzeichnet, dass sie in hart verkleideten Kapseln enthalten ist.

3. Trockene pulverige Allergenzusammensetzung nach Anspruch 1 oder 2, dadurch gekennzeichnet, dass das Gewichtsverhältnis des Bindemittels zum aktiven Prinzip des Allergens von 1:1 bis 50.000:1 beträgt.

4. Trockene pulverige Zusammensetzung nach einem beliebigen der vorhergehenden Ansprüchen, dadurch gekennzeichnet, dass die Partikelabmessungen des Pulvers von 2 bis 100 micron betragen.

5. Verfahren zur Herstellung der trockenen pulverigen Allergenzusammensetzung nach einem beliebigen der vorhergehenden Patentansprüche, bestehend in der Extraktion des aktiven Prinzips des Allergens durch Wasserextraktion, in der Mischung des aktiven Prinzips des Allergens mit dem Bindemittel und Mahlung des Gemisches zu einer gleichmässigen Partikelabmessung, dadurch gekennzeichnet, dass die Mischung des aktiven Prinzips mit dem Bindemittel durch Zugabe der wässerigen Lösung des aktiven Prinzips des Allergens dem Bindemittel in solcher Menge, dass eine feuchte Masse erhalten wird, durch Mischung und Homogenisierung der feuchten, so erhaltenen Masse durch wiederholte Durchgänge durch ein erstes Sieb mit vorzugsweise Maschenabmessungen von 500 micron, durch Trocknung der so erhaltenen Masse, Behandlung der pulverigen Masse zu einem feinen Pulver durch wiederholte Durchgänge durch Siebe mit immer kleineren Maschenabmessungen durchgeführt wird, wobei das letzte Sieb Maschen mit 50 micron Maschenabmessungen besitzt, so dass ein Pulver erhalten wird, welches das gleichmässig in dem Bindemittel verteilte Allergen enthält.

6. Verfahren zur Herstellung der trockenen pulverigen Allergenzusammensetzung nach Anspruch 5, dadurch gekennzeichnet, dass die Mischung des aktiven Prinzips mit dem Bindemittel durch Zugabe des Bindemittels dem Wasser, Zugabe der so erhaltenen Lösung dem aktiven Prinzip des Allergens in der Form einer Lösung oder eines entwässerten Pulvers, Rühren des Gemisches, bis eine einwandfreie Homogenisierung erhalten wird, Entwässerung der homogenen Lösung durch Gefrieren-Trocknen, Mahlung der entwässerten Masse und Pulverisierung der gemahlten Masse entweder mittels wiederholter Durchgänge durch Siebe mit zunehmend kleineren Maschenabmessungen oder mittels Mikronisierung erhalten wird.

**Revendications**

1. Composition en poudre sèche allergénique, comprenant un mélange du principe actif de l'agent allergenique et ane ou plusieures matières de support, caractérisée en ce que la dilution du principe actif de l'agent allergénique dans la matière de support est uniforme et homogène, en mélangeant en solution ou en condition baignée le principe actif avec la matière de support.

2. Composition allergénique en poudre sèche, selon la revendication 1, caractérisée en ce que ladite composition est contenue dans des capsules pourvues d'enveloppes ou ques dures.

3. Composition allergénique en poudre sèche selon la revendication 1, caractérisée en ce que le rapport en poids entre la matière de support et le principe actif de l'agent allergénique est compris entre 1:1 et 5000:1.

4. Composition allergénique en poudre sèche selon l'une quelconque des revendications précédentes, caractérisée en ce que la dimension des particules de la poudre est comprise entre 2 et 100 micromètres.

5. Méthode pour la préparation de la composition allergénique en poudre sèche selon l'une quelconque des revendications précédentes, consistant dans l'extraction du principe actif de l'agent allergénique au moyen d'une extraction aqueouse, le mélangement du principe actif avec la matière de support et le broyage du mélange pour obtenir une dimension uniforme des particules, caractérisée en ce que le mélangement du principe actif avec la matière de support est réalisé en additionnant la solution aqueouse du principe actif de l'agent allergénique à la matière de support dans une quantité telle à former une masse humide, en mélangeant et homogénéisant la masse humide ainsi obtenue au moyen de passages répétés à travers d'un premier tamis ayant préférablement une dimension des mailles de 500 micromètres, en dessicant la masse homogénéisée ainsi obtenue, réduisant la masse de poudre dans une poudre fine au moyen de passages répétés à travers des tamis ayant une dimension des mailles progressivément décroissante, le dernier tanis ayant une dimension des mailles de 50 micromètres, de manière qu'on obtient une poudre conténant l'agent allergénique dispersé d'une manière uniforme dans la matière de

6. Méthode pour la préparation de la composition allergénique en poudre sèche selon la revendication 5, caractérisée en ce que le mélangement du principe actif avec la matière de support est réalisé en additionnant la matière de support à de l'eau, additionnant à la solution ainsi obtenue le principe actif de l'agent allergénique sous la forme d'une solution ou d'une poudre déshydratée, agitant le mélange jusqu'à l'obtention d'une homogénéité parfaite, déshydratant la solution homogène au moyen de congélation-dessication, broyant la masse sèche et réduisant dans une poudre la masse broyée soit au moyen de passages répétés à travers des tamis ayant une dimension des mailles progressivément décrosissante soit au moyen de micronisation.